# EUROPEAN PATENT SPECIFICATION

(11) **EP 2 521 453 B1**
(45) Date of publication and mention of the grant of the patent: **11.05.2016**
(21) Application number: 11732265.1
(22) Date of filing: 10.01.2011
(51) Int. Cl.: A01N 45/00, A61K 31/59, A61P 25/06, A61P 43/00

(54) **COMBINATIONS OF VITAMIN D AND A STATIN FOR THE TREATMENT OF MIGRAINE HEADACHES**
ZUSAMMENSETZUNGEN AUS VITAMIN D UND EINEM STATIN ZUR BEHANDLUNG VON MIGRÄNE
COMPOSITIONS CONTENANT DU VITAMIN D ET UNE STATINE DESTINÉES AU TRAITEMENT DES CÉPHALÉES MIGRAINEUSES

(30) Priority: 15.10.2010 US 393680 P; 08.01.2010 US 293379 P
(43) Date of publication of application: 14.11.2012
(73) Proprietor: Beth Israel Deaconess Medical Center, Boston, MA 02215 (US)
(72) Inventor: BURSTEIN, Rami, Chestnut Hill MA 02467 (US); BUETTNER, Catherine, Boston MA 02118 (US)
(74) Representative: Lahrtz, Fritz
(86) International application number: PCT/US2011/020643
(87) International publication number: WO 2011/085293

(56) References cited:
- US-A1- 2008 161 393
- US-A1- 2009 163 452
- THYS-JACOBS, S: "Vitamin D and Calcium in Menstrual Migraine", HEADACHE: THE JOURNAL OF HEAD AND FACE PAIN, vol. 34, no. 9, 18 May 2005 (2005-05-18), pages 544-546, XP055061101, DOI: 10.1111/j.1526-4610.1994.hed3409544.x
- MEDEIROS ET AL.: 'Simvastatin for Migraine Prevention.' HEADACHE IN BRAZIL vol. 47, no. 6, June 2007, pages 855 - 856
- THYS-DAVIS.: 'Alleviation of Migraines with Therapeutic Vitamin D and Calcium. (Synopsis) I Headache' THE JOURNAL OF HEAD AND FACE PAIN November 1994, pages 590 - 592
- IHS CLINICAL TRIALS COMMITTEE: "Guidelines for controlled trials of drugs in migraine: second edition", CEPHALGIA, vol. 20, 1 January 2000 (2000-01-01), pages 765-786,

## Description

### Background of the Invention

Migraine headache is associated with dural and meningeal vascular dilatation and perivascular inflammation. The vasculopathy of migraines is thought to reflect endothelial dysfunction, a disorder of endothelial activation and impaired vascular reactivity. Endothelial activation gives rise to inflammation and thrombosis, while impaired vascular reactivity reduces bioavailability of vasodilators and increases the level of endothelium-derived contracting factors. Migraine sufferers may also experience abnormal muscle tenderness during migraine episodes. Muscle tenderness may be chronic, may appear several hours before the onset of a migraine attack, or may appear several hours after the onset of migraine.

Current migraine therapies are inadequate for treating migraine sufferers who are intolerant to such therapies or for those who suffer from frequent, severe, or intractable migraine pain. While several classes of prophylactic medications are available to treat this population, many of the existing migraine therapies are minimally effective or are not well tolerated. In addition, many individuals who experience migraine are at increased risk for cardiovascular events, which are not addressed by current prophylactics.

For example, simvastatin has been described in the reduction of migraine frequency (Medeiros et al., Headache 2007, 47(6), 855-856), while a combination therapy of vitamin D and a source of calcium has been implicated in the alleviation of migraine (Thys-Jacobs, S., Headache 1994, 34(9), 544-546; Thys-Jacobs, S., Headache 1994 34(10), 590-592.

Thus, there exists a need in the art for therapies that treat, ameliorate, and/or prevent migraines, associated symptoms, and cardiovascular risk, such as stroke.

### Summary of the Invention

The present invention is related to compositions for use in methods for treating or reducing the likelihood of a migraine, reducing the frequency of migraines, and ameliorating the symptoms of a migraine.

In a first aspect, the invention features statin and vitamin D for use in a method of treating or reducing the likelihood of a migraine in a subject (e.g., a human) in need thereof. The method includes administering to a subject (i) a statin and (ii) vitamin D, wherein a statin and vitamin D are administered in amounts and for a duration that together are sufficient to treat or reduce the likelihood of a migraine in a subject.

In a second aspect, the disclosure describes a method of ameliorating the symptoms of a migraine in a subject (e.g., a human) in need thereof. The method includes administering to a subject (i) a statin or analogs thereof and (ii) vitamin D or analogs thereof, wherein a statin or analogs thereof and vitamin D or analogs thereof are administered in amounts and for a duration that together are sufficient to ameliorate the symptoms of a migraine in a subject.

In a third aspect, the disclosure describes a method of reducing the frequency or duration of migraines in a subject (e.g., a human) in need thereof. The method includes administering to a subject (i) a statin or analogs thereof and (ii) vitamin D or analogs thereof, wherein a statin or analogs thereof and vitamin D or analogs thereof are to be administered in amounts and for a duration that together are sufficient to reduce the frequency or duration of migraines in a subject.

In one embodiment of the first, second, third, or fourth aspect, the statin is atorvastatin; cerivastatin; fluvastatin; lovastatin; rosuvastatin; acitemate; amlodipine/atorvastatin; BAY102987; BAY X 2678; BB476; bervastatin; BMY21950; BMY22089; colestolone; CP83101; dalvastatin; DMP565; ezetimibe/simvastatin; glenvastatin; L659699; L669262; mevastatin; nicotinic acid/lovastatin; nicotinic acid/simvastatin; P882222; P882284; PD134965; PD135022; pitavastatin; rosuvastatin; RP61969; S2468; SC37111; SC45355; simvastatin; SQ33600; SR12813; SR45023A; U20685; and U88156. In another embodiment, vitamin D is doxercalciferol, calcitriol, α-calcidol, seocalcitol, calcipotriol, maxacalcitol, falecalcitriol, paricalcitol, alfacalcidol, calcifediol, cholecalciferol, dihydrotachysterol, ergosterol, ergocalciferol, 1α,2-dihydroxyvitamin D₄, 1α,24-dihydroxyvitamin D₂, 1α,25-dihydroxyvitamin D₂, 1α,25-dihydroxyvitamin D₄, or 1α,24,25-dihydroxyvitamin D₂.

In another embodiment of the first, second or third aspect, the statin is simvastatin or the vitamin D is ergocalciferol and/or cholecalciferol. In a particular embodiment, the statin is simvastatin and the vitamin D is ergocalciferol or cholecalciferol.

In one embodiment of any of the above aspects, a statin and vitamin D are for administration simultaneously or sequentially.

In a fourth aspect, the invention features a pharmaceutical composition that includes (i) a statin, (ii) vitamin D, and (iii) a pharmaceutically acceptable carrier, wherein a statin and vitamin D are present in an amount that, when administered to a subject, are sufficient to treat or reduce the likelihood of a migraine in a subject.

In a fifth aspect, the invention features a pharmaceutical composition that includes (i) a statin, (ii) vitamin D , and (iii) a pharmaceutically acceptable carrier, wherein a statin and vitamin D are present in an amount that, when administered to a subject, are sufficient to ameliorate the symptoms of a migraine in a subject.

In a sixth aspect, the invention features a pharmaceutical composition that includes (i) a statin, (ii) vitamin D, and (iii) a pharmaceutically acceptable carrier, wherein a statin and vitamin D are present in an amount that, when administered to a subject, are sufficient to reduce the frequency or duration of migraines in a subject.

In one embodiment of the fourth, fifth or sixth aspect, a statin and vitamin D are formulated in a single composition. The pharmaceutical compositions may be formulated for oral administration or systemic administration. Such pharmaceutical compositions may be formulated for inhalation or for topical administration.

In another embodiment of the fourth, fifth or sixth aspect, the statin is simvastatin or the vitamin D is ergocalciferol and/or cholecalciferol. In a particular embodiment, the statin is simvastatin and the vitamin D is ergocalciferol or cholecalciferol.

In one embodiment of any of the above aspects, an additional therapeutic agent is to be administered to a subject or present in a pharmaceutical composition. The additional therapeutic agent may be an analgesic (e.g., a non-steroidal anti-inflammatory agent), a β-blocked, a calcium channel blocker an antiemetic, a serotonin receptor agonist, a barbiturate, an antidepressant (e.g., a tricyclic antidepressant), an ergot alkaloid, a steroid, an anxiolytic, an amphetamine, a NOS inhibitor, a narcotic, or an anticonvulsant (e.g., valproic acid).

By "an amount sufficient" is meant the amount of a compound or therapeutic agent, alone or in combination with another compound or therapeutic regimen, required to treat or ameliorate a disorder, such as a migraine headache, in a clinically relevant manner. A sufficient amount of an active compound or therapeutic agent used to practice the present invention for therapeutic treatment of, e.g., a migraine headache varies depending upon the manner of administration, age, and general health of the subject. Ultimately, the medical practitioner prescribing such treatment will decide the appropriate amount and dosage regimen. Additionally, an effective amount may be an amount of compound in the combination of the invention that is safe and efficacious in the treatment of a subject having a disorder, such as a migraine headache, over each agent alone as determined and approved.

By "migraine" is meant a subset of headaches characterized by unusually severe, unilateral, throbbing head pain that often includes additional symptoms described herein. Migraine is meant to include, for example, migraine without aura (e.g., common migraine), migraine with aura (e.g., classical migraine), migraine with typical aura, migraine with prolonged aura, migraine without headache, hemiplegic migraine (e.g., familial hemiplegic migraine), basilar migraine (e.g., basilar artery migraine), carotidynia, abdominal migraine (e.g., periodic syndrome), hormonal migraine (e.g., pregnancy-induced migraine), exertion migraine, migraine with acute onset aura, ophthalmoplegic migraine, status migrainous, transformed migraine, retinal migraine, nocturnal migraine, childhood periodic syndromes that may be precursors to or associated with migraine, benign paroxysmal vertigo of childhood, alternating hemiplegia of childhood, and migrainous infarction.

By "pharmaceutically acceptable carrier" is meant a carrier that is physiologically acceptable to the treated subject while retaining the therapeutic properties of the compound (e.g., a statin or analogs thereof and vitamin D or analogs thereof) with which it is administered. One exemplary pharmaceutically acceptable carrier substance is physiological saline. Other physiologically acceptable carriers and their formulations are known to one skilled in the art.

By "pharmaceutically acceptable salts" is meant salts that are, within the scope of sound medical judgment, suitable for use in contact with the tissues of a subject without undue toxicity, irritation, or allergic response, and are commensurate with a reasonable benefit/risk ratio. Pharmaceutically acceptable salts are well known in the art. The salts can be prepared *in situ* during the final isolation and purification of the therapeutic compounds of the invention or separately by reacting the free base function with a suitable organic acid. Representative acid addition salts include, e.g., acetate, ascorbate, aspartate, benzoate, citrate, digluconate, fumarate, glucoheptonate, glycerophosphate, hemisulfate, heptonate, hexanoate, hydrobromide, hydrochloride, hydroiodide, lactate, malate, maleate, malonate, mesylate, oxalate, phosphate, succinate, sulfate, tartrate, thiocyanate, valerate salts. Representative alkali or alkaline earth metal salts include sodium, lithium, potassium, calcium, magnesium, as well as nontoxic ammonium, quaternary ammonium, and amine cations, includingammonium, tetramethylammonium, tetraethylammonium, methylamine, dimethylamine, trimethylamine, triethylamine, and ethylamine.

By "reducing the likelihood of' is meant reducing the severity, the frequency, and/or the duration of a migraine or symptoms thereof. Reducing the likelihood of migraines is synonymous with migraine prophylaxis or the chronic treatment of migraines.

By "sequentially administering" is meant administering the therapeutic agents at separately staggered times (e.g., within 1 hour, 4 hours, 12 hours, 1 day, 7 days, or within 14 days of each other). Thus, the therapeutic agents (e.g., a statin and vitamin D) can be sequentially administered such that the beneficial pharmaceutical effect of the statin and vitamin D are realized by the subject at substantially the same time.

By "simultaneously administering" is meant administering the therapeutic agents substantially concurrently. The term "simultaneously administering" encompasses not only administering the two or more therapeutic agents (e.g., a statin and vitamin D) in a single pharmaceutical dosage form, but also the administration of each active agent in its own separate pharmaceutical dosage formulation. Where separate dosage formulations are used, the therapeutic agents can be administered at essentially the same time.

By "statin" is meant a compound capable of inhibiting the enzyme HMG-CoA reductase. Members of the statin family include naturally occurring and synthetic molecules, e.g., atorvastatin; cerivastatin; fluvastatin; lovastatin; rosuvastatin; acitemate; amlodipine/atorvastatin; BAY102987; BAY X 2678; BB476; bervastatin; BMY21950; BMY22089; colestolone; CP83101; dalvastatin; DMP565; simvastatin; ezetimibe/simvastatin; glenvastatin; L659699; L669262; mevastatin; nicotinic acid/lovastatin; nicotinic acid/simvastatin; P882222; P882284; PD134965; PD135022; pitavastatin; pravastatin; rosuvastatin; RP61969; S2468; SC37111; SC45355; simvastatin; SQ33600; SR12813; SR45023A; U20685; and U88156.

By "subject" is meant any animal, e.g., a mammal (e.g., a human). A subject who is being treated for a migraine is one who has been diagnosed by a medical practitioner as having such a condition or being predisposed to such a condition. Subjects of the invention may also include those that do not presently have a migraine. Diagnosis may be performed by any suitable means, such as those described herein. One in the art will understand that subjects treated using the compositions of the present invention may have been subjected to standard tests or may have been identified, without examination, as one at high risk due to the presence of one or more risk factors, such as age, genetics, or family history.

By "systemic administration" is meant any non-dermal route of administration and specifically excludes topical and transdermal routes of administration.

By "therapeutic agent" is meant any agent that produces a healing, curative, stabilizing, or ameliorative effect.

By "treating" or "ameliorating" is meant treating or ameliorating a condition or symptom(s) of the condition (e.g., migraine headache) before or after its onset. Symptoms of migraines include, e.g., severe headache, nausea, muscle tenderness, abdominal pain, visual aura, sensory hyperexcitability (e.g., photophobia, phonophobia, or osmophobia), blurred vision, nasal congestion, diarrhea, polyuria, pallor, sweating, localized edema of the scalp or face, scalp tenderness, prominence of a vein or artery in the temple, stiffness or tenderness of the neck, impairment of concentration or mood, vertigo, lightheadedness, fatigue, depression, and euphoria. As compared with an equivalent untreated control, such amelioration or degree of treatment is at least 5%, 10%, 20%, 30%, 40%, 50%, 60%, 70%, 80%, 90%, 95%, 99%, or 100%, as measured by any standard technique.

By "vitamin D" is meant a compound that binds to the vitamin D receptor (VDR). Vitamin D compounds and analogs thereof include vitamin D₁ (ergocalciferol with lumisterol); vitamin D₂ (ergocalciferol); vitamin D₃ (cholecalciferol); vitamin D₄ (22-dihydroergocalciferol); vitamin D₅ (sitocalciferol); doxercalciferol; calcitriol, α-calcidol; seocalcitol; calcipotriol; maxacalcitol; falecalcitriol; paricalcitol; alfacalcidol; calcifediol; dihydrotachysterol; ergosterol; 1α,24-dihydroxyvitamin D₄; 1α,24-dihydroxyvitamin D₂; 1α,25-dihydroxyvitamin D₂; 1α,25-dihydroxyvitamin D₄; or 1α,24,25-dihydroxyvitamin D₂.

Other features and advantages of the invention will be apparent from the following Detailed Description and the claims.

### Detailed Description of the Invention

We describe compositions for treating or reducing the likelihood of a migraine, reducing the frequency of migraines, reducing the duration of migraines, and ameliorating the symptoms of a migraine.

### Therapeutic Agents

The co-administration of a statin and vitamin D may be used for the treatment of any subject that has been diagnosed with migraines, for the prevention of migraines, for reducing the frequency of migraines, or for the amelioration of symptoms (e.g., headaches) associated with migraines.

### Statins

Statins may be used as therapeutic agents in the compositions and methods of the invention. Statins of the invention include, e.g., atorvastatin; cerivastatin; fluvastatin; lovastatin; rosuvastatin; acitemate; amlodipine/atorvastatin; BAY102987; BAY X 2678; BB476; bervastatin; BMY21950; BMY22089; colestolone; CP83101; dalvastatin; DMP565; simvastatin; ezetimibe/simvastatin; glenvastatin; L659699; L669262; mevastatin; nicotinic acid/lovastatin; nicotinic acid/simvastatin; P882222; P882284; PD 134965; PD135022; pitavastatin; pravastatin; rosuvastatin; RP61969; S2468; SC37111; SC45355; simvastatin; SQ33600; SR12813; SR45023A; U20685; and U88156.

Additional statins and analogs thereof are described, for example, in U.S. Patent Nos. 3,983,140; 4,231,938; 4,282,155; 4,293,496; 4,294,926; 4,319,039; 4,343,814; 4,346,227; 4,351,844; 4,361,515; 4,376,863; 4,444,784; 4,448,784; 4,448,979; 4,450,171; 4,503,072; 4,517,373; 4,661,483; 4,668,699; 4,681,893; 4,719,229; 4,738,982; 4,739,073; 4,766,145; 4,782,084; 4,804,770; 4,841,074; 4,847,306; 4,857,546; 4,857,547; 4,940,727; 4,946,864; 5,001,148; 5,006,530; 5,075,311; 5,112,857; 5,116,870; 5,120,848; 5,166,364; 5,173,487; 5,177,080; 5,273,995; 5,276,021; 5,369,123; 5,385,932; 5,502,199; 5,763,414; 5,877,208; and 6,541,511, and U.S. Patent Application Publication Nos. 2002/0013334; 2002/0028826; 2002/0061901; and 2002/0094977.

### Vitamin D

Vitamin D may be used as therapeutic agents in the compositions and methods of the invention. Vitamin D compounds and analogs thereof include, e.g., vitamin D₁ (ergocalciferol with lumisterol); vitamin D₂ (ergocalciferol); vitamin D₃ (cholecalciferol); vitamin D₄ (22-dihydroergocalciferol); vitamin D₅ (sitocalciferol); doxercalciferol; calcitriol, α-calcidol; seocalcitol; calcipotriol; maxacalcitol; falecalcitriol; paricalcitol; alfacalcidol; calcifediol; dihydrotachysterol; ergosterol; 1α,24-dihydroxyvitamin D₄; 1α,24-dihydroxyvitamin D₂; 1α,25-dihydroxyvitamin D₂; 1α,25-dihydroxyvitamin D₄; or 1α,24,25-dihydroxyvitamin D₂.

Additional vitamin D compounds and analogs thereof are described, for example, in U.S. Patent Nos. 5,194,248; 6,103,709; 6,329,357; 6,706,725; 6,831,106; 7,205,420; 7,241,909; 7,259,143; 7,312,249; and 7,538,098.

### Additional Therapeutic Agents

If desired, the subject may also receive additional therapeutic regimens. For example, therapeutic agents may be administered with the therapeutic agents (e.g., statin and vitamin D) described herein at concentrations known to be effective for such therapeutic agents. Particularly useful agents include those that treat or ameliorate symptoms of a migraine. Exemplary agents are analgesics (e.g., non-steroidal anti-inflammatory drugs; acetaminophen; or opiates); β-blockers (e.g., alprenolol; bucindolol; carteolol; carvedilol; labetalol; nadolol; penbutolol; pindolol; propranolol; timolol; acebutolol; atenolol; betaxolol; bisoprolol; celiprolol; esmolol; metoprolol; nebivolol; and butaxamine); calcium channel blockers (e.g., mibefradil; bepridil; fluspirilene; diltiazem; verapamil; gallopamil; amlodipine; aranidipine; azelnidipine; barnidipine; benidipine; cilnidipine; clevidipine; efonidipine; felodipine; lacidipine; lercanidipine; manidipine; nicardipine; nifedipine; nilvadipine; nimodipine; nisoldipine; nitrendipine; and pranidipine); antiemetics (e.g., 5-HT₃ receptor antagonists (e.g., dolasetron and ondansetron); dopamine antagonists (e.g., metoclopramide); or antihistamines); serotonin receptor agonists (e.g., azapirones and triptans); barbiturates; antidepressants (e.g., monoamine oxidase inhibitors; tricyclic antidepressants (e.g., amitriptyline; butriptyline; clomipramine; desipramine; dosulepin; doxepin; imipramine; lofepramine; nortriptyline; protriptyline; and trimipramine); tetracyclic antidepressants; selective serotonin reuptake inhibitors; and serotoninnorepinephrine reuptake inhibitors); ergot alkaloids; steroids; anxiolytics (e.g., benzodiazepines (e.g., alprazolam; chlordiazepoxide; clonazepam; diazepam; or lorazepam); azapirones (e.g., buspirone and tandospirone); and hydroxyzine); amphetamines; nitric oxide synthase (NOS) inhibitors; narcotics; or anticonvulsants (e.g., carbamazepine; and valproic acid).

The therapeutic agents of the invention may be admixed with additional active or inert ingredients, e.g., in conventional pharmaceutically acceptable carriers. A pharmaceutical carrier can be any compatible, non-toxic substance suitable for the administration of the compositions of the present invention to a subject (e.g., a human). Pharmaceutically acceptable carriers include, for example, water, saline, buffers, and other compounds described, for example, in the Merck Index, Merck & Co., Rahway, New Jersey.

In addition to the administration of therapeutic agents, additional therapeutic regimen may involve, e.g., a modification to the lifestyle of the subject being treated. Such lifestyle changes may be helpful to control migraines and include, e.g., increased exercise and avoidance of migraine triggers (e.g., food triggers or stress). Other lifestyle changes may include, e.g., weight loss, nutritional supplementation (e.g., glucosamine or chondroitin), the use of orthotics, and acupuncture.

### Formulation

The co-administration of a statin and vitamin D may be used for the treatment of any subject that has been diagnosed with migraines, for the prevention of migraines, for reducing the frequency of migraines, or for the amelioration of symptoms (e.g., headaches) associated with migraines.

Any of the therapeutic agents employed according to the present invention may be contained in any appropriate amount in any suitable carrier substance and is generally present in an amount of 1-95% by weight of the total weight of the composition. Therapeutic formulations are prepared using standard methods known in the art by mixing the active ingredient having the desired degree of purity with physiologically acceptable carriers, excipients, or stabilizers in the form of lyophilized formulations or aqueous solutions (see, e.g., Remington's Pharmaceutical Sciences, 20th edition, Ed. A. Gennaro, 2000, Lippincott, Williams & Wilkins, Philadelphia, PA). Acceptable carriers include, e.g., saline; buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagines, arginine, or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, PLURONICS™, or PEG.

The formulations of the present invention may contain a pharmaceutically acceptable salt (e.g., sodium chloride) at physiological concentrations. The formulations of the invention can contain a pharmaceutically acceptable preservative. In some embodiments, the preservative concentration ranges from 0.1 to 2.0% v/v. Suitable preservatives include those known in the pharmaceutical arts. Benzyl alcohol, phenol, m-cresol, methylparaben, and propylparaben are preferred preservatives. The formulations of the invention can include a pharmaceutically acceptable surfactant. Surfactants may include, e.g., non-ionic detergents, Tween-20®, and pluronic acid (F68). Suitable surfactant concentrations are, e.g., 0.005 to 0.02%.

The composition may be provided in a dosage form that is, e.g., suitable for the oral (e.g., sublingual), parenteral (e.g., intravenous or intramuscular), rectal, cutaneous, nasal, vaginal, inhalant, skin (e.g., via a skin patch), or ocular administration route. Thus, the composition may be in the form of, e.g., tablets, capsules, pills, powders, granulates, suspensions, emulsions, solutions, gels (e.g., hydrogels), pastes, ointments, creams, plasters, drenches, osmotic delivery devices, suppositories, enemas, injectables, implants, sprays, or aerosols

For intranasal administration or administration by inhalation, the compounds of the invention may be delivered in the form of, e.g., a solution or suspension from a pump spray container that is squeezed or pumped by the patient or as an aerosol spray from a pressurized container or a nebulizer with the use of a suitable propellant, e.g., dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base, such as lactose or starch.

Where sustained-release administration is desired, microencapsulation of the therapeutic agents of the present invention is contemplated. The sustained-release formulations may include those developed using poly-lactic-coglycolic acid (PLGA) polymer. The degradation products of PLGA, lactic and glycolic acid, can be cleared quickly from the human body. Moreover, the degradability of this polymer can be adjusted from months to years depending on its molecular weight and composition.

Each agent of the present invention may be formulated in a variety of ways that are known in the art. Desirably, the therapeutic agents are formulated together for the simultaneous or near simultaneous administration of the therapeutic agents. Such co-formulated compositions can include the two agents formulated together in the same, e.g., pill, capsule, or liquid. It is to be understood that, when referring to the formulation of such combinations, the formulation technology employed is also useful for the formulation of the individual agents of the combination, as well as other combinations of the invention. By using different formulation strategies for different agents, the pharmacokinetic profiles for each agent can be suitably matched.

### Dosages

Generally, when administered to a human, the dosage of any of the therapeutic agents of the invention will depend on the nature of the agent and can readily be determined by one skilled in the art. Typically, such dosage is normally 0.001 mg to 2000 mg per day, desirably 1 mg to 1000 mg per day, and more desirably 5 mg to 500 mg per day.

Administration of each drug, alone or in combination, can be one to four times dally for one day to one year and may even be for the life of the subject. Chronic, long-term administration will be required in some cases.

Appropriate dosages of compounds used in the methods described herein depend on several factors, including the administration method, the severity of the disorder (e.g., migraine), and the age, weight, and health of the subject to be treated. Additionally, pharmacogenomic information (e.g., the effect of genotype on the pharmacokinetic, pharmacodynamic, or efficacy profile of a therapeutic) about a particular subject may affect the dosage used.

From the foregoing description, it will be apparent that variations and modifications may be made to the invention described herein to adopt it to various usages and conditions.

From the foregoing description, one skilled in the art can easily ascertain the essential characteristics of this invention; can make various changes and modifications of the invention to adapt it to various usages and conditions.

## Claims

1. A (i) statin and (ii) vitamin D for use in a method of treating or reducing the likelihood of a migraine in a subject in need thereof, wherein the method comprises administration of said statin and said vitamin D in combination to said subject to treat or reduce the likelihood of a migraine in said subject.

2. A (i) statin and (ii) vitamin D for use in the method according to claim 1, wherein the (i) statin and (ii) the vitamin D ameliorate the symptoms of a migraine in the subject.

3. A (i) statin and (ii) vitamin D for use in the method according to claim 1, wherein the statin and (ii) the vitamin D reduce the frequency or duration of migraines in the subject.

4. The (i) statin and (ii) vitamin D for use in the method according to any one of claims 1-3, wherein the statin is simvastatin and the vitamin D is cholecalciferol.

5. The (i) statin and (ii) vitamin D for use in the method according to any one of claims 1-3, wherein said statin is atorvastatin; cerivastatin; fluvastatin; lovastatin; rosuvastatin; acitemate; amlodipine/atorvastatin; BAY102987; BAYX 2678; BB476; bervastatin; BMY21950; BMY22089; colestolone; CP83101; dalvastatin; DMP565; ezetimibe/simvastatin; glenvastatin; L659699; L669262; mevastatin; nicotinic acid/lovastatin; nicotinic acid/simvastatin; P882222; P882284; PD134965; PD135022; pitavastatin; rosuvastatin, RP61969; S2468; SC37111; SC45355; simvastatin; SQ33600; SR12813; SR45023A; U20685; or U88156, preferably wherein said statin is simvastatin.

6. The (i) statin and (ii) vitamin D for use in the method according to any one of claims 1-3, wherein said vitamin D is doxercalciferol, calcitriol, α-ccilcidol, seocalcitol, calcipotriol, maxacalcitol, falecalcitriol, paricalcitol, alfacalcidol, calcifediol, cholecalciferol, dihydrotachysterol, ergosterol, ergocalciferol, 1α,2-dihydroxyvitamin D₄, 1α,24-dihydroxyvitamin D₂, 1α,25-dihydroxyvitamin D₂, 1α,25-dihydroxyvitamin D₄, or 1α,24,25-dihydroxyvitamin D₂, preferably wherein said vitamin D is cholecalciferol or ergocalciferol, more preferably wherein said statin is simvastatin.

7. The (i) statin and (ii) vitamin D for use in the method according to any one of claims 1-3, wherein the method further comprises administering an additional therapeutic agent to said subject, preferably wherein said additional therapeutic agent is an analgesic, a β-blocker, a calcium channel blocker, an antiemetic, a serotonin receptor agonist, a barbiturate, an antidepressant, an ergot alkaloid, a steroid, an anxiolytic, an amphetamine, a NOS inhibitor, a narcotic, or an anticonvulsant, more preferably wherein said analgesic is a non-steroidal anti-inflammatory agent, or wherein said antidepressant is a tricyclic antidepressant, or wherein said anticonvulsant is valproic acid.

8. The (i) statin and (ii) vitamin D for use in the method according to any one of claims 1-7, wherein said subject is a human subject.

9. A pharmaceutical composition comprising (i) a statin, (ii) vitamin D, and (iii) a pharmaceutically acceptable carrier, wherein said composition is for use in treating or reducing the likelihood of a migraine in said subject.

10. The pharmaceutical composition according to claim 9, wherein said composition is for use in ameliorating the symptoms of a migraine in said subject.

11. The pharmaceutical composition according to claim 9, wherein said composition is for use in reducing the frequency or duration of migraines in said subject

12. The pharmaceutical composition of any one of claims 10-11, wherein said statin and said vitamin D are formulated in a single composition.

13. The pharmaceutical composition of any one of claims 10-12, wherein said statin and said vitamin D are formulated for oral administration, systemic administration, inhalation, or topical application, preferably wherein said statin is atorvastatin; cerivastatin; fluvastatin; lovastatin; rosuvastatin; acitemate; amlodipine/atorvastatin; BAY102987; BAY X 2678; BB476; bervastatin; BMY21950; BMY22089; colestolone; CP83101; dalvastatin; DMP565; ezetimibe/simvastatin; glenvastatin; L659699; L669262; mevastatin; nicotinic acid/lovastatin; nicotinic acid/simvastatin; P882222; P882284; PD134965; PD135022; pitavastatin; rosuvastatin; RP61969; S2468; SC37111; SC45355; simvastatin; SQ33600; SR12813; SR45023A; U20685; or U88156, more preferably wherein said statin is simvastatin.

14. The pharmaceutical composition of any one of claims 10-13, wherein said vitamin D is doxercalciferol, calcitriol, α-calcidol, seocalcitol, calcipotriol, maxacalcitol, falecalcitriol, paricalcitol, alfacalcidol, calcifediol, cholecalciferol, dihydrotachysterol, ergosterol, ergocalciferol, 1α,2-dihydroxyvitamin D₄, 1α,24-dihydroxyvitamin D₂, 1α;25-dihydroxyvitamin D₂, 1α,25-dihydroxyvitamin D₄, or 1α,24,25-dihydroxyvitamin D₂, preferable wherein said vitamin D is cholecalciferol or ergocalciferol, more preferably wherein said vitamin D is cholecalciferol, or wherein said vitamin D is ergocalciferol, most preferably wherein said statin is simvastatin.

15. The pharmaceutical composition of any one of claims 10-14, wherein said composition further comprises an additional therapeutic agent, preferably wherein said additional therapeutic agent is an analgesic, a β-blocker, a calcium channel blocker, an antiemetic, a serotonin receptor agonist, a barbiturate, an antidepressant, an ergot alkaloid, a steroid, an anxiolytic, an amphetamine, a NOS inhibitor, a narcotic, or an anticonvulsant, more preferably wherein said analgesic is a non-steroïdal anti-inflammatory agent, or wherein said antidepressant is a tricyclic antidepressant, or wherein said anticonvulsant is valproic acid.

## Patentansprüche

1. (i) Statin und (ii) Vitamin D zur Verwendung in einem Verfahren zur Behandlung oder Reduzierung der Wahrscheinlichkeit einer Migräne in einem Subjekt, das einen Bedarf daran hat, wobei das Verfahren die Verabreichung dieses Statins und dieses Vitamins D in Kombination an dieses Individuum umfasst, um die Wahrscheinlichkeit einer Migräne in diesem Individuum zu behandeln oder zu reduzieren.

2. (i) Statin und (ii) Vitamin D zur Verwendung in dem Verfahren gemäß Anspruch 1, wobei das (i) Statin und (ii) das Vitamin D die Symptome einer Migräne in dem Subjekt verbessern.

3. (i) Statin und (ii) Vitamin D zur Verwendung in dem Verfahren gemäß Anspruch 1, wobei das (i) Statin und (ii) das Vitamin D die Häufigkeit oder Dauer von Migränen in dem Subjekt reduzieren.

4. (i) Statin und (ii) Vitamin D zur Verwendung in dem Verfahren gemäß einem beliebigen der Ansprüche 1-3, wobei das Statin Simvastatin und das Vitamin D Cholecalciferol ist.

5. (i) Statin und (ii) Vitamin D zur Verwendung in dem Verfahren gemäß einem beliebigen der Ansprüche 1-3, wobei dieses Statin Atorvastatin, Cerivastatin, Fluvastatin, Lovastatin, Rosuvastatin, Acitemat, Amlodipin/Atorvastatin, BAY102987, BAY X 2678, BB476, Bervastatin, BMY21950, BMY22089, Colestolon, CP83101, Dalvastatin, DMP565, Ezetimib/Simvastatin, Glenvastatin, L659699, L669262, Mevastatin, Nikotinsäure/Lovastatin, Nikotinsäure/Simvastatin, P882222, P882284, PD134965, PD135022, Pitavastatin, Rosuvastatin, RP61969, S2468, SC37111, SC45355, Simvastatin, SQ33600, SR12813, SR45023A, U20685 oder U88156 ist, bevorzugt wobei dieses Statin Simvastatin ist.

6. (i) Statin und (ii) Vitamin D zur Verwendung in dem Verfahren gemäß einem beliebigen der Ansprüche 1-3, wobei dieses Vitamin D Doxercalciferol, Calcitriol, α-Calcidol, Seocalcitol, Calcipotriol, Maxacalcitol, Falecalcitriol, Paricalcitol, Alfacalcidol, Calcifediol, Cholecalciferol, Dihydrotachysterol, Ergosterol, Ergocalciferol, 1α,2-Dihydroxyvitamin D₄, 1α,24-Dihydroxyvitamin D₂, 1α,25-Dihydroxyvitamin D₂, 1α,25-Dihydroxyvitamin D₄ oder 1α,24,25-Dihydroxyvitamin D₂ ist, bevorzugt wobei dieses Vitamin D Cholecalciferol oder Ergocalciferol ist, stärker bevorzugt wobei dieses Statin Simvastatin ist.

7. (i) Statin und (ii) Vitamin D zur Verwendung in dem Verfahren gemäß einem beliebigen der Ansprüche 1-3, wobei das Verfahren weiterhin die Verabreichung eines zusätzlichen therapeutischen Mittels an dieses Subjekt umfasst, bevorzugt wobei dieses zusätzliche therapeutische Mittel ein Analgetikum, ein β-Blocker, ein Kalziumkanalblocker, ein Antibrechmittel, ein Serotonin-Rezeptoragonist, ein Barbiturat, ein Antidepressivum, ein Ergot-Alkaloid, ein Steroid, ein Anxiolytikum, ein Amphetamin, ein NOS-Inhibitor, ein Narkotikum oder ein antikonvulsives Mittel ist, stärker bevorzugt wobei dieses Analgetikum ein nicht-steroidales antiinflammatorisches Mittel ist oder wobei dieses Antidepressivum ein tricyclisches Antidepressivum ist oder wobei dieses antikonvulsive Mittel Valproinsäure ist.

8. (i) Statin und (ii) Vitamin D zur Verwendung in dem Verfahren gemäß einem beliebigen der Ansprüche 1-7, wobei dieses Subjekt ein menschliches Subjekt ist.

9. Pharmazeutische Zusammensetzung, umfassend (i) ein Statin, (ii) Vitamin D und (iii) einen pharmazeutisch verträglichen Träger, wobei diese Zusammensetzung zur Verwendung bei der Behandlung oder Reduktion der Wahrscheinlichkeit einer Migräne in diesem Subjekt ist.

10. Pharmazeutische Zusammensetzung gemäß Anspruch 9, wobei diese Zusammensetzung zur Verwendung bei der Verbesserung der Symptome einer Migräne in diesem Subjekt ist.

11. Pharmazeutische Zusammensetzung gemäß Anspruch 9, wobei diese Zusammensetzung zur Verwendung bei der Reduktion der Häufigkeit oder Dauer von Migränen in diesem Subjekt ist.

12. Pharmazeutische Zusammensetzung gemäß einem beliebigen der Ansprüche 10-11, wobei dieses Statin und dieses Vitamin D in einer einzigen Zusammensetzung formuliert sind.

13. Pharmazeutische Zusammensetzung gemäß einem beliebigen der Ansprüche 10-12, wobei dieses Statin und dieses Vitamin D zur oralen Verabreichung, systemischen Verabreichung, Inhalation oder topischen Verabreichung formuliert sind, bevorzugt wobei dieses Statin Atorvastatin, Cerivastatin, Fluvastatin, Lovastatin, Rosuvastatin, Acitemat, Amlodipin/Atorvastatin, BAY102987, BAY X 2678, BB476, Bervastatin, BMY21950, BMY22089, Colestolon, CP83101, Dalvastatin, DMP565, Ezetimib/Simvastatin, Glenvastatin, L659699, L669262, Mevastatin, Nikotinsäure/Lovastatin, Nikotinsäure/Simvastatin, P882222, P882284, PD134965, PD135022, Pitavastatin, Rosuvastatin, RP61969, S2468, SC37111, SC45355, Simvastatin, SQ33600, SR12813, SR45023A, U20685 oder U88156 ist, stärker bevorzugt wobei dieses Statin Simvastatin ist.

14. Pharmazeutische Zusammensetzung gemäß einem beliebigen der Ansprüche 10-13, wobei dieses Vitamin D Doxercalciferol, Calcitriol, α-Calcidol, Seocalcitol, Calcipotriol, Maxacalcitol, Falecalcitriol, Paricalcitol, Alfacalcidol, Calcifediol, Cholecalciferol, Dihydrotachysterol, Ergosterol, Ergocalciferol, 1α,2-Dihydroxyvitamin D₄, 1α,24-Dihydroxyvitamin D₂, 1α,25-Dihydroxyvitamin D₂, 1α,25-Dihydroxyvitamin D₄ oder 1α,24,25-Dihydroxyvitamin D₂ ist, bevorzugt wobei dieses Vitamin D Cholecalciferol oder Ergocalciferol ist, stärker bevorzugt wobei dieses Vitamin D Cholecalciferol ist oder wobei dieses Vitamin D Ergocalciferol ist, am stärksten bevorzugt wobei dieses Statin Simvastatin ist.

15. Pharmazeutische Zusammensetzung gemäß einem beliebigen der Ansprüche 10-14, wobei diese Zusammensetzung weiterhin ein zusätzliches therapeutisches Mittel umfasst, bevorzugt wobei dieses zusätzliche therapeutische Mittel ein Analgetikum, ein β-Blocker, ein Kalziumkanalblocker, ein Antibrechmittel, ein Serotonin-Rezeptoragonist, ein Barbiturat, ein Antidepressivum, ein Ergot-Alkaloid, ein Steroid, ein Anxiolytikum, ein Amphetamin, ein NOS-Inhibitor, ein Narkotikum oder ein antikonvulsives Mittel ist, stärker bevorzugt wobei dieses Analgetikum ein nicht-steroidales antiinflammatorisches Mittel ist oder wobei dieses Antidepressivum ein tricyclisches Antidepressivum ist oder wobei dieses antikonvulsive Mittel Valproinsäure ist.

## Revendications

1. (i) Statine et (ii) vitamine D pour utilisation dans un procédé de traitement ou de réduction du risque de migraine chez un sujet qui le nécessite, le procédé comprenant l'administration de ladite statine et de ladite vitamine D en association audit sujet pour traiter ou réduire le risque de migraine chez ledit sujet.

2. (i) Statine et (ii) vitamine D pour utilisation dans le procédé selon la revendication 1, (i) la statine et (ii) la vitamine D améliorant les symptômes d'une migraine chez le sujet.

3. (i) Statine et (ii) vitamine D pour utilisation dans le procédé selon la revendication 1, la statine et (ii) la vitamine D réduisant la fréquence ou la durée des migraines chez le sujet.

4. (i) Statine et (ii) vitamine D pour utilisation dans le procédé selon l'une quelconque des revendications 1 à 3, la statine étant de la simvastatine et la vitamine D étant du cholécalciférol.

5. (i) Statine et (ii) vitamine D pour utilisation dans le procédé selon l'une quelconque des revendications 1 à 3, ladite statine étant de l'atorvastatine ; de la cérivastatine ; de la fluvastatine ; de la lovastatine ; de la rosuvastatine ; de l'acitémate ; de l'amlodipine/atorvastatine ; du BAY102987 ; du BAY X 2678 ; du BB476 ; de la bervastatine ; du BMY21950 ; du BMY22089 ; de la colestolone ; du CP83101 ; de la dalvastatine ; du DMP565 ; de l'ézétimibe/simvastatine ; de la glenvastatine ; du L659699 ; du L669262 ; de la mévastatine ; de l'acide nicotinique/lovastatine ; de l'acide nicotinique/simvastatine ; du P882222 ; du P882284 ; du PD134965 ; du PD135022 ; de la pitavastatine ; de la rosuvastatine ; du RP61969 ; du S2468 ; du SC37111 ; du SC45355 ; de la simvastatine ; du SQ33600 ; du SR12813 ; du SR45023A ; du U20685 ; ou du U88156, de préférence ladite statine étant de la simvastatine.

6. (i) Statine et (ii) vitamine D pour utilisation dans le procédé selon l'une quelconque des revendications 1 à 3, ladite vitamine D étant du doxercalciférol, du calcitriol, de l'α-calcidol, du séocalcitol, du calcipotriol, du maxacalcitol, du falécalcitriol, du paricalcitol, de l'alfacalcidol, du calcifédiol, du cholécalciférol, du dihydrotachystérol, de l'ergostérol, de l'ergocalciférol, de la 1α,2-dihydroxyvitamine D₄, de la 1α,24-dihydroxyvitamine D₂, de la 1α,25-dihydroxyvitamine D₂, de la 1α,25-dihydroxyvitamine D₄ ou de 1α,24,25-dihydroxyvitamine D₂, de préférence ladite vitamine D étant du cholécalciférol ou de l'ergocalciférol, plus préférablement ladite statine étant de la simvastatine.

7. (i) Statine et (ii) vitamine D pour utilisation dans le procédé selon l'une quelconque des revendications 1 à 3, le procédé comprenant en outre l'administration d'un agent thérapeutique additionnel audit sujet, de préférence ledit agent thérapeutique additionnel étant un analgésique, un β-bloquant, un bloqueur des canaux calciques, un antiémétique, un agoniste des récepteurs de la sérotonine, un barbiturique, un antidépresseur, un alcaloïde de l'ergot, un stéroïde, un anxiolytique, une amphétamine, un inhibiteur de NOS, un narcotique ou un anticonvulsivant, plus préférablement ledit analgésique étant un anti-inflammatoire non stéroïdien, ou ledit antidépresseur étant un antidépresseur tricyclique, ou ledit anticonvulsivant étant de l'acide valproïque.

8. (i) Statine et (ii) vitamine D pour utilisation dans le procédé selon l'une quelconque des revendications 1 à 7, ledit sujet étant un sujet humain.

9. Composition pharmaceutique comprenant (i) une statine, (ii) de la vitamine D et (iii) un vecteur pharmaceutiquement acceptable, ladite composition étant destinée à une utilisation dans le traitement ou la réduction du risque de migraine chez ledit sujet.

10. Composition pharmaceutique selon la revendication 9, ladite composition étant destinée à une utilisation dans l'amélioration des symptômes d'une migraine chez ledit sujet.

11. Composition pharmaceutique selon la revendication 9, ladite composition étant destinée à une utilisation dans la réduction de la fréquence ou de la durée des migraines chez ledit sujet.

12. Composition pharmaceutique selon l'une quelconque des revendications 10 à 11, dans laquelle ladite statine et ladite vitamine D sont préparées sous la forme d'une seule composition.

13. Composition pharmaceutique selon l'une quelconque des revendications 10 à 12, dans laquelle ladite statine et ladite vitamine D sont préparées pour une administration orale, une administration systémique, une inhalation ou une application topique, de préférence dans laquelle ladite statine est de l'atorvastatine ; de la cérivastatine ; de la fluvastatine ; de la lovastatine ; de la rosuvastatine ; de l'acitémate ; de l'amlodipine/atorvastatine ; du BAY102987 ; du BAY X 2678 ; du BB476 ; de la bervastatine ; du BMY21950 ; du BMY22089 ; de la colestolone ; du CP83101 ; de la dalvastatine ; du DMP565 ; de l'ézétimibe/simvastatine ; de la glenvastatine ; du L659699 ; du L669262 ; de la mévastatine ; de l'acide nicotinique/lovastatine ; de l'acide nicotinique/simvastatine ; du P882222 ; du P882284 ; du PD134965 ; du PD135022 ; de la pitavastatine ; de la rosuvastatine ; du RP61969 ; du S2468 ; du SC37111 ; du SC45355 ; de la simvastatine ; du SQ33600 ; du SR12813 ; du SR45023A ; du U20685 ; ou du U88156, plus préférablement dans laquelle ladite statine est de la simvastatine.

14. Composition pharmaceutique selon l'une quelconque des revendications 10 à 13, dans laquelle ladite vitamine D est du doxercalciférol, du calcitriol, de l'α-calcidol, du séocalcitol, du calcipotriol, du maxacalcitol, du falécalcitriol, du paricalcitol, de l'alfacalcidol, du calcifédiol, du cholécalciférol, du dihydrotachystérol, de l'ergostérol, de l'ergocalciférol, de la 1α,2-dihydroxyvitamine D₄, de la 1α,24-dihydroxyvitamine D₂, de la 1α,25-dihydroxyvitamine D₂, de la 1α,25-dihydroxyvitamine D₄ ou de 1α,24,25-dihydroxyvitamine D₂, de préférence ladite vitamine D étant du cholécalciférol ou de l'ergocalciférol, plus préférablement dans laquelle ladite vitamine D est du cholécalciférol, ou dans laquelle ladite vitamine D est de l'ergocalciférol, de manière préférée entre toutes dans laquelle ladite statine est de la simvastatine.

15. Composition pharmaceutique selon l'une quelconque des revendications 10 à 14, ladite composition comprenant en outre un agent thérapeutique additionnel, de préférence ledit agent thérapeutique additionnel étant un analgésique, un β-bloquant, un bloqueur des canaux calciques, un antiémétique, un agoniste des récepteurs de la sérotonine, un barbiturique, un antidépresseur, un alcaloïde de l'ergot, un stéroïde, un anxiolytique, une amphétamine, un inhibiteur de NOS, un narcotique ou un anticonvulsivant, plus préférablement ledit analgésique étant un anti-inflammatoire non stéroïdien, ou ledit antidépresseur étant un antidépresseur tricyclique, ou ledit anticonvulsivant étant de l'acide valproïque.
